(19) Europäisches Patentamt
European Patent Office
Office européen des brevets

(11) Veröffentlichungsnummer: **0 040 398**
**B1**

(12) # EUROPÄISCHE PATENTSCHRIFT

(45) Veröffentlichungstag der Patentschrift: **16.01.85**

(51) Int. Cl.[4]: **C 07 D 277/68**, C 07 D 277/84, C 07 D 513/06 // (C07D513/06, 277/00, 221/00)

(21) Anmeldenummer: **81103687.0**

(22) Anmeldetag: **13.05.81**

(54) **Verfahren zur Herstellung von in 3-Stellung substituierten 2(3H)-Benzthiazolonen oder 2(3H)-Naphthothiazolonen.**

(30) Priorität: **16.05.80 DE 3018668**

(43) Veröffentlichungstag der Anmeldung: **25.11.81 Patentblatt 81/47**

(45) Bekanntmachung des Hinweises auf die Patenterteilung: **16.01.85 Patentblatt 85/3**

(84) Benannte Vertragsstaaten: **BE CH DE FR GB IT LI**

(56) Entgegenhaltungen:
**DE - A - 2 924 712**

**BERICHTE DER DEUTSCHEN CHEMISCHEN GESELLSCHAFT, Band 13, 1880 A.W. HOFMANN "Ueber eine Reihe aromatischer, den Senfölen und Sulfocyanaten isomerer Basen" Seiten 8 bis 22**
**JOURNAL OF THE CHEMICAL SOCIETY 1962 F. KURZER et al. "Heterocyclic Compounds from Urea Derivatives. Part III. Synthesis and Cyclisation of Isothioureas Derived from *o*-Aminothiophenol and Diarylcarbodi-imides" Seiten 230 bis 236**
**Patents Abstracts of Japan Band 2, Nr. 76, 16. Juni 1978 Seite 978C78**

(73) Patentinhaber: **HOECHST AKTIENGESELLSCHAFT, Postfach 80 03 20, D-6230 Frankfurt am Main 80 (DE)**

(72) Erfinder: **Papenfuhs, Theodor, Dr., Heinrich-Bleicher-Strasse 40, D-6000 Frankfurt am Main 50 (DE)**

**EP 0 040 398 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung liegt auf dem technischen Gebiet der Zwischenprodukte und betrifft insbesondere ein verbessertes Verfahren zur Herstellung von in 3-Stellung substituierten 2(3H)-Benzthiazolonen oder 2(3H)-Naphthothiazolonen.

In 3-Stellung substituierte 2(3H)-Benzthiazolone sind technisch wichtige Zwischenprodukte, die beispielsweise aus der deutschen Offenlegungsschrift 2 924 712 bekannt sind. Die Synthese dieser Verbindungsklasse ist technisch bisher nicht befriedigend gelöst. Die bekannten Verfahren basieren auf technisch nicht zugänglichen Ausgangsprodukten oder auf solchen Ausgangsprodukten, die nur unter Anwendung ökologisch und apparativ aufwendiger Wege zugänglich sind. Im einzelnen sind folgende Herstellungsmöglichkeiten bekannt geworden:

a) Alkylierung von 2-Alkylthiobenzthiazolen mit nachfolgender Hydrolyse;
b) Thermische Zersetzung von 3-substituierten 2-Nitrosoamino-benzthiazolen;
c) Umsetzung von N-monosubstituierten o-Mercapto-anilinen mit Phosgen;
d) Alkylierung von Benzthiazolonen;
e) Thermische Umlagerung von 2-Alkoxy-benzthiazolen.

Die obigen Verfahrensweisen a) bis c) besitzen nur wissenschaftliches Interesse, da sie bezüglich der gewählten Verfahrensschritte ökologisch bedenklich sind und teilweise nur schlechte Ausbeuten liefern. Die Verfahrensweisen d) und e) gehen dagegen von 2-Chlor-benzthiazolen als Ausgangsverbindungen aus, für die allerdings kein technisch realisierbarer Herstellungsweg bekannt geworden ist, so dass diese Verfahren keine technische Verwertungsmöglichkeit zulassen.

Es bestand somit ein erhebliches Interesse an einem allgemein anwendbaren, technisch realisierbaren und verbesserten Verfahren zur Herstellung von in 3-Stellung substituierten 2(3H)-Benzthiazolonen.

Mit der vorliegenden Erfindung wurde nunmehr ein Verfahren zur Herstellung von in 3-Stellung substituierten 2(3H)-Benzthiazolonen oder von 2(3H)-Naphthothiazolonen gefunden, das dadurch gekennzeichnet ist, dass man eine in 3-Stellung substituierte 2(3H)-Iminobenzthiazolin- bzw. -naphthothiazolin-Verbindung mit einem Alkali- oder Erdalkalihydroxyd in einem alkalibeständigen Löse- oder Verdünnungsmittel in Abwesenheit von Wasser oder unter weitgehendem Ausschluss von Wasser behandelt und das sich so bildende Alkali- oder Erdalkalisalz der am Stickstoff substituierten ortho-Mercapto-N-phenyl- bzw. -naphthylharnstoff-Verbindung, gegebenenfalls ohne deren Zwischenisolierung, durch Behandlung mit einer Säure zur in 3-Stellung substituierten 2(3H)-Benzthiazolon- bzw. 2(3H)-Naphthothiazolon-Verbindung cyclisiert.

Im nachfolgenden Text wird bei den in der erfindungsgemässen Reaktion eingesetzten Ausgangsverbindungen, auftretenden Zwischenverbindungen und den Endprodukten der Einfachheit halber nur von den Benzolderivaten gesprochen. Ihre Erwähnung soll automatisch die Naphthalinderivate einschliessen.

Die erfindungsgemässe Reaktion läuft nach folgendem Schema:

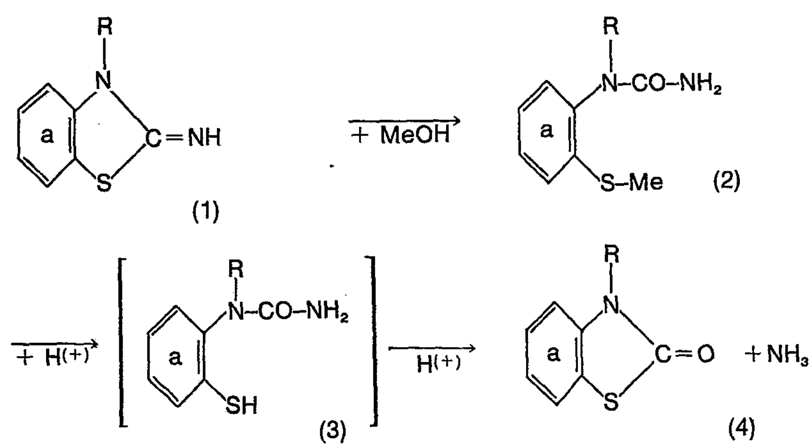

(1) (2) (3) (4)

(In den Formeln (1) bis (4) steht Me für das Äquivalent eines Alkalimetalls oder Erdalkalimetalls, und der Benzolkern a kann weitere Substituenten tragen; der Rest R ist ein gegebenenfalls substituierter Alkyl-, Cycloalkyl oder Arylrest, wobei R mit dem Benzolkern oder Naphthalinkern verbunden sein kann. Das bei der Cyclisierung frei werdende Ammoniak wird durch die Säure als Ammoniumion gebunden).

Die zur Ringöffnung in die Reaktion eingesetzten Alkali- oder Erdalkalihydroxide MeOH sind bevorzugt Barium-, Calcium- und Kaliumhydroxid, insbesondere jedoch Natriumhydroxid. Die zur Cyclisierung der Zwischenverbindungen der Formel (2) in die Reaktion eingesetzten Säuren sind vorzugsweise Mineralsäuren, wie Salzsäure, Schwefelsäure und Phosphorsäure.

Alkalibeständige Löse- oder Verdünnungsmittel, die wasserfrei sein sollten und in denen erfindungsgemäss die Ringspaltung der in 3-Stellung substituierten 2(3H)-Iminobenzthiazoline, gegebenenfalls auch die Cyclisierung der daraus entstehenden Mercaptophenylharnstoffe, durchgeführt wird, sind insbesondere ein- oder mehrwertige

aliphatische Alkohole, wie beispielsweise Alkanole von 1 bis 6 C-Atomen, Alkanglykole von 2 bis 5 C-Atomen, Alkantriole von 3 bis 8 C-Atomen, höhermolekulare Alkanpolyole und niedere Monoalkyläther der genannten Glykole, Triole und Polyole.

Es war äusserst überraschend, dass die technisch gut zugänglichen in 3-Stellung substituierten 2(3H)-Iminobenzthiazolin-Verbindungen (siehe die DE-OS 2 834 852) auf einfache Weise und in hoher Ausbeute und Reinheit unter Verwendung technisch leicht zugänglicher und billiger Chemikalien, die keine Abwasserprobleme aufwerfen, in die in 3-Stellung substituierten 2(3H)-Benzthiazolon-Verbindungen übergeführt werden können. Aus Chem. Ber. 13, 20 (1880) ist nämlich bekannt, dass die artverwandten 2-Aminobenzthiazole bei der Behandlung mit geschmolzenem Alkali unter Zerstörung des heterocyclischen Ringes irreversibel zu o-Amino-thiophenolaten und Ammoniak sowie Alkalicarbonaten gespalten werden. Mit der vorliegenden Erfindung wurde dagegen eine schonende Ringöffnung erreicht. Bei dem erfindungsgemässen Verfahren entweicht praktisch kein Ammoniak, und man erhält in hoher, oft nahezu quantitativer Ausbeute als Zwischenverbindungen die Alkali- oder Erdalkalisalze der entsprechenden o-Mercaptophenylharnstoffe (s. obige Formel (2). Diese stabilen Mercaptide der Formel (2) lassen sich dann direkt oder nach deren Zwischenisolierung durch eine Säurebehandlung in die in 3-Stellung substituierten 2(3H)-Benzthiazolone überführen.

Diese säurekatalytische Cyclisierung der Verbindungen der Formel (2) bzw. (3) zu den in 3-Stellung substituierten 2(3H)-Benzthiazolonen der Formel (4) ist zwar als Analogreaktion aus J. Chem. Soc. 1962, 230, bekannt, jedoch blieb einer Cyclisierungsreaktion dieser Art, übertragen auf die obigen Verbindungen entsprechend der Formel (2) oder (3), bisher die technische Ausführung verschlossen, weil eine technische Herstellung der Ausgangsverbindungen der Formel (3) bisher nicht möglich war.

Mit der Auffindung des neuen Verfahrens zur Herstellung der Schlüsselverbindung der Formel (2) bzw. (3) ist es nunmehr möglich, in einfacher, technisch vorteilhafter Weise aus leicht zugänglichen Vorprodukten in 3-Stellung substituierte 2(3H)-Benzthiazolon-Verbindungen herzustellen.

Von den als Ausgangsverbindungen dienenden in 3-Stellung substituierten 2(3H)-Iminobenzthiazolin-Verbindungen kommen bevorzugt solche in Frage, die in der deutschen Offenlegungsschrift 2 834 852 beschrieben sind. Besonders bevorzugt werden nach dem erfindungsgemässen Verfahren die Benzthiazolone der allgemeinen Formel (4a)

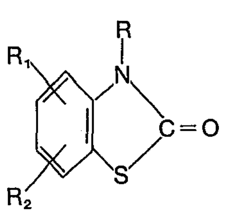

(4a)

hergestellt, in welcher $R_1$ und $R_2$ gleich oder verschieden voneinander sind und jedes ein Wasserstoffatom, eine Alkylgruppe, bevorzugt niedere Alkylgruppe, eine Alkoxygruppe, bevorzugt eine niedere Alkoxygruppe, ein Halogenatom, bevorzugt ein Fluor- oder Chloratom, eine Hydroxy-, Nitro- oder Sulfogruppe oder beide zusammen mit dem Kern a einen Naphthalinkern bedeuten und R ein Alkylrest ist, der substituiert sein kann, oder ein Cycloalkylrest oder ein Aralkyl- oder Arylrest ist, wobei dieser Rest in o-Stellung des Benzolkernes a mit diesem verbunden sein kann, bevorzugt aber einen Alkylrest von 1 bis 5 C-Atomen darstellt, der durch ein oder zwei Substituenten aus der Gruppe Vinyl, durch Methyl, Äthyl, Methoxy, Äthoxy und/oder Chlor ggf. substituiertes Phenyl, Amino, Dimethylamino, Diäthylamino, Hydroxy, niederes Alkoxy und Piperidino substituiert sein kann, oder bevorzugt ein Cyclohexylrest ist oder ein Phenylrest ist, der durch Substituenten aus der Gruppe Methyl, Äthyl, Hydroxy, Methoxy, Äthoxy und Chlor substituiert sein kann, oder R einen Propylenrest bedeutet, der im Benzolkern a in o-Stellung zum Stickstoff ringgeschlossen ist. Dabei wird erfindungsgemäss von 2-Iminobenzthiazolinen der allgemeinen Formel (1a)

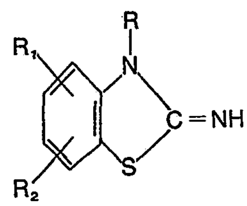

(1a)

in welcher R, $R_1$ und $R_2$ die obengenannten Bedeutungen haben, ausgegangen.

Das erfindungsgemässe Verfahren der Ringspaltung der in 3-Stellung substituierten 2(3H)-Iminobenzthiazoline zu den entsprechenden, am Stickstoffatom substituierten o-Mercaptophenylharnstoffen, wie beispielsweise der Formel (2), soll in wasserfreiem Medium durchgeführt werden, um hohe Ausbeuten zu gewährleisten. Die Umsetzung kann zwar auch in Gegenwart von geringen Mengen Wasser, die in den Löse- oder Verdünnungsmitteln vorhanden sein können, durchgeführt werden, jedoch verringert sich hierdurch die Ausbeute und gegebenenfalls Reinheit des Verfahrensproduktes. Die Forderung in dem erfindungsgemässen Verfahren geht deshalb dahin, die Verfahrensweise der Ringspaltung der in 3-Stellung substituierten 2(3H)-Iminobenzthiazoline unter weitgehendem Ausschluss von Wasser, d.h. vorteilhaft in Anwesenheit von höchstens 2,5 Gew.-%, vorzugsweise höchstens 0,5 Gew.-%, Wasser in der Reaktionsmischung und bezogen auf die Reaktionsmischung, auszuführen.

Die Reaktionstemperatur des erfindungsgemässen Verfahrensschrittes zur Herstellung der Verbindungen der Formel (2) kann zweckmässig im Bereich zwischen 80 und 200 °C gewählt werden; vorzugsweise arbeitet man bei einer Temperatur zwischen 120 und 160 °C. Bei Verwendung von niedrigsiedenden Löse- oder Verdünnungsmitteln, wie beispielsweise Alkanolen von 1 bis 3

C-Atomen oder niederen Glykol-monoalkyläthern, arbeitet man deshalb im geschlossenen System unter Druck.

Das bei der Ringspaltungsreaktion verwendete Alkali- oder Erdalkalihydroxid wird vorzugsweise in zumindest stöchiometrischer Menge eingesetzt. Zur Vermeidung von Nebenreaktionen hat sich ein Überschuss von einer 1- bis 4-fach, vorzugsweise einer 2- bis 3-fach molaren Menge Alkali- oder Erdalkalihydroxid als günstig erwiesen.

Die erfindungsgemäss hergestellten Alkali- bzw. Erdalkalimetallsalze der ortho-Mercaptophenylharnstoffe werden anschliessend, gegebenenfalls nach Abtrennung aus dem Reaktionsmedium durch Entfernung des Löse- oder Verdünnungsmittels, durch Säure in die ortho-Thiophenol-harnstoff-Verbindung übergeführt. Hierzu sind äquivalente Mengen an Säure erforderlich. Die anschliessende Cyclisierung dieser Thiophenolharnstoffe zu den in 3-Stellung substituierten 2(3H)-Benzthiazolonen kann sowohl mit katalytischen als auch mit überschüssigen Mengen an Säure erfolgen. Die Cyclisierungsreaktion kann in einem Löse- oder Verdünnungsmittel durchgeführt werden, vorteilhaft jedoch in dem wässrigen Medium der angewandten Säure. Die Cyclisierungsreaktion erfolgt zweckmässig bei einer Temperatur zwischen 40 und 100 °C, bevorzugt 60 und 90 °C; sie ist in kurzer Zeit beendet.

Das erfindungsgemässe Verfahren kann in der Weise durchgeführt werden, dass man eine in 3-Stellung substituierte 2(3H)-Iminobenzthiazolin-Verbindung, beispielsweise der allgemeinen Formel (1) oder (1a), in einem wasserfreien oder praktisch wasserfreien Lösemittel, wie Äthanol, Isobutanol, 1,2-Dihydroxypropan oder 1,3-Dihydroxypropan, bevorzugt Äthylenglykol, Glycerin, Äthylenglykol-monomethyläther oder Äthylenglykol-monoäthyläther, mit dem festen Alkali- oder Erdalkalihydroxid, bevorzugt Natriumhydroxid, unter Rühren auf eine Temperatur zwischen 120 und 160 °C erhitzt und innerhalb dieses Temperaturbereiches die Reaktion mehrere Stunden weiterführt. Die vollständige Umsetzung kann durch chromatographische Prüfung auf Ausgangsverbindungen kontrolliert werden. Während dieser Ringöffnungsreaktion entweicht praktisch kein Ammoniak. Das Alkali- oder Erdalkalisalz der gebildeten N-substituierten o-Mercaptophenylharnstoff-Verbindung scheidet sich kristallin aus dem Reaktionsgemisch ab und kann durch Filtration von der gegebenenfalls recyclisierbaren Mutterlauge abgetrennt und der anschliessenden Cyclisierungsreaktion zugeführt werden. Das gebildete Salz der N-substituierten o-Mercaptophenylharnstoff-Verbindung kann jedoch auch im Reaktionsmedium belassen werden und direkt durch Zugabe einer mindestens stöchiometrischen Menge, bezogen auf das eingesetzte Alkali- oder Erdalkalihydroxid, an Mineralsäure in die freie N-substituierte o-Mercaptophenylharnstoff-Verbindung überführt werden, die sich, beispielsweise nach Zugabe von Wasser, durch Filtration isolieren lässt. Infolge der Instabilität dieser freien N-substituierten o-Mercaptophenylharnstoff-Verbindungen ist es jedoch vorteilhaft, diese oder deren Salz direkt im sauren Medium durch kurzzeitiges Erhitzen zur gewünschten in 3-Stellung substituierten 2(3H)-Benzthiazolon-Verbindung zu cyclisieren. In diesem letzteren Falle erfolgt die Isolierung des Endproduktes entweder durch Filtration aus dem eingesetzten Lösemittel, gegebenenfalls nach weiterer Verdünnung mit Wasser, oder durch Abblasen des Lösemittels und nachfolgende mechanische Trennung der erhaltenen Suspension, beispielsweise mittels Filterpresse, Separator oder Zentrifuge. – Geht man zur Cyclisierung von dem isolierten Salz der N-substituierten o-Mercaptophenylharnstoff-Verbindung bzw. der isolierten freien Mercaptoverbindung aus, so wird dieses in Wasser, das ein weiteres Löse- oder Verdünnungsmittel enthalten kann, gelöst oder suspendiert; nach Zugabe von Säure bis zu einem sauren pH-Wert wird dieses Gemisch zweckmässig auf 40 bis 100 °C, vorzugsweise 60 bis 90 °C, erhitzt.

In einigen Fällen kann auch so verfahren werden, dass man nach der Ringöffnung das Löse- oder Verdünnungsmittel aus der alkalischen Reaktionsmischung abdestilliert oder mit Wasserdampf abbläst und das hierbei erhaltene Salz der o-Mercaptophenyl-harnstoff-Verbindung bzw. deren wässrige Lösung mit Säure, vorzugsweise wässriger Säure, bis zum sauren pH-Bereich versetzt und sodann im oben angegebenen Temperaturbereich erhitzt.

Das erfindungsgemässe Verfahren geht von technisch vorteilhaft zugänglichen Ausgangsverbindungen aus und liefert in hohen Ausbeuten und Qualitäten in technisch einfachen Verfahrensschritten und in ökologisch günstiger Weise die entsprechenden in 3-Stellung substituierten 2(3H)-Benzthiazolon-Verbindungen.

Die nachfolgenden Beispiele dienen zur Erläuterung der Erfindung. Die Teile sind Gewichtsteile, die Prozentangaben beziehen sich auf Gewichtsprozente, sofern nichts anderes vermerkt. Gewichtsteile stehen im Verhältnis zu Volumenteilen wie Kilogramm zu Liter.

Beispiel 1

Eine Mischung aus 164 Teilen 2-Imino-3-methylbenzthiazolin, 150 Teilen Natriumhydroxid und 300 Teilen Äthylenglykol wird 6 Stunden bei 130 bis 140 °C gerührt, sodann auf 80 °C abgekühlt und unter Rühren in 3000 Teile Eiswasser einlaufen lassen. Es wird weitergerührt, und nach 30 Minuten werden 5 Teile Aktivkohle zugesetzt; es wird 15 Minuten weitergerührt, die Lösung filtriert, das Filtrat mit 10%iger Salzsäure auf einen pH-Wert von 5 bis 6 gestellt und der ausgefallene N-Methyl-N-(2-mercapto-phenyl)-harnstoff durch Filtration isoliert. Das feuchte Produkt wird unverzüglich in 700 Teile 10%iger Salzsäure eingetragen und 15 Minuten bei 90 °C gerührt. Das ausfallende 3-Methyl-2(3H)-benzthiazolon der Formel

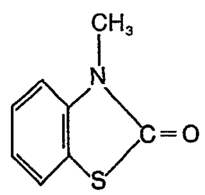

wird nach dem Abkühlen durch Filtration isoliert, mit Wasser gewaschen und getrocknet. Man erhält 150 Teile analysenreines Produkt mit einem Schmelzpunkt von 73 bis 75 °C (entsprechend 90,9% d.Th., bezogen auf das eingesetzte 2-Imino-3-methyl-benzthiazolin).

Verwendet man anstelle von Natriumhydroxid eine entsprechende Menge an Kaliumhydroxid oder Bariumhydroxid und arbeitet in der angegebenen Verfahrensweise, so erhält man das 3-Methyl-2(3H)-benzthiazolon in praktisch gleich guter Ausbeute und Qualität.

Beispiel 2

Eine Mischung aus 138 Teilen 3-Phenyl-6,7-benzo-2(3H)-benzthiazolin, 50 Teilen Natriumhydroxid und 150 Teilen Glycerin wird 12 Stunden bei 150 °C gerührt, sodann langsam in 2000 Teile Eiswasser einlaufen lassen. Die dabei erhaltene Lösung des Natriumsalzes des N-Phenyl-N-(1-mercapto-naphth-2-yl)-harnstoffs der Formel

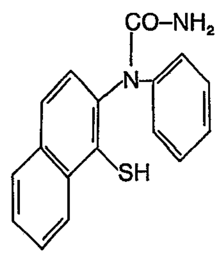

wird nach Zusatz von 2,5 Teilen Aktivkohle geklärt. Das Filtrat wird mit 250 Teilen 30%iger Salzsäure versetzt, sodann unter Rühren während 15 Minuten auf 85–90 °C erhitzt, anschliessend auf 20 °C abgekühlt; das ausgefallene 3-Phenyl-6,7-benzo-2-(3H)-benzthiazolon der Formel

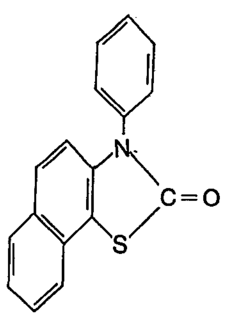

wird abfiltriert, mit Wasser gewaschen und getrocknet. Man erhält 130,9 Teile analysenreines Produkt mit einem Schmelzpunkt von 143 bis 145 °C (entsprechend 94,5% d.Th.). – Man verfährt in der hier angegebenen Verfahrensweise, versetzt jedoch das den Mercaptonaphthylharnstoff enthaltende Filtrat anstelle mit der Salzsäure mit einer äquivalenten Menge an Schwefel- oder Phosphorsäure; man erhält das Naphthothiazolon in gleich guter Ausbeute und Reinheit. – Man verfährt in der hier angegebenen Verfahrensweise, ersetzt jedoch das Lösemittel Glycerin durch die gleiche Menge an 1,2-Dihydroxypropan oder 1,3-Dihydroxypropan; man erhält das Naphtho-thiazolon in praktisch gleich guter Ausbeute und Qualität.

Beispiel 3

Eine Mischung aus 106,25 Teilen 3-Äthyl-6-chlor-2-iminobenzthiazolin, 60 Teilen Natriumhydroxid und 150 Teilen Isobutanol wird in einem Autoklaven 9 Stunden bei 140 °C gerührt. Nach dem Abkühlen wird die erhaltene Suspension des Natriumsalzes des N-Äthyl-N-(4-chlor-2-mercapto-phenyl)-harnstoffs filtriert. Der Filterrückstand wird mit Isobutanol gewaschen und bei reduziertem Druck getrocknet. Man erhält 116,4 Teile des Natriumsalzes dieser Thioverbindung (entsprechend 92,2% d.Th.). Es wird in 400 Teile 10%ige Salzsäure eingetragen, die erhaltene Suspension 30 Minuten bei 80 °C erwärmt, wobei anfangs Lösung des Produktes eintritt und nachfolgend das gebildete 3-Äthyl-6-chlor-2(3H)-benzthiazolon farblos ausfällt. Es wird nach Abkühlen filtriert, mit Wasser gewaschen und getrocknet. Man erhält 95,5 Teile des Produktes, das praktisch analysenrein ist und einen Schmelzpunkt von 112 bis 114 °C besitzt; die Ausbeute ist dementsprechend 89,5% d.Th.

Beispiel 4

Man verfährt in der im Beispiel 3 anfangs angegebenen Verfahrensweise, trocknet jedoch nicht das isolierte Natriumsalz des Mercaptophenylharnstoffes, sondern lässt es isobutanolfeucht und trägt es in die angegebene Menge Salzsäure ein; nach 30minütigem Erwärmen auf 80 °C kühlt man ab, filtriert und erhält nach Waschen mit Wasser und Trocknen 97 Teile 3-Äthyl-6-chlor-2(3H)-benzthiazolon mit einem Schmelzpunkt von 111 bis 113 °C entsprechend einer Ausbeute von 90,0% d.Th.

Beispiel 5

Verfährt man in der im Beispiel 3 oder im Beispiel 4 angegebenen Verfahrensweise, verwendet jedoch als Löse- oder Verdünnungsmittel anstelle von Isobutanol eine gleiche Menge an n-Butanol oder Äthanol oder Äthylenglykol-monomethyläther, so erhält man das 3-Äthyl-6-chlor-2(3H)-benzthiazolon in praktisch gleich guter Qualität und Ausbeute.

Beispiel 6

Eine Mischung aus 95,0 Teilen 3,4-Trimethylen-2-iminobenzthiazolin, 100 Teilen Äthylenglykol und 60 Teilen Natriumhydroxid wird 4 Stunden unter Rühren bei 160 bis 165 °C erhitzt; anschliessend wird das Reaktionsgemisch unter Rühren auf 20 bis 30 °C abgekühlt, das ausgefallene Natriumsalz des 8-Mercapto-1,2,3,4-tetrahydrochinolin-N-carbonamids der Formel

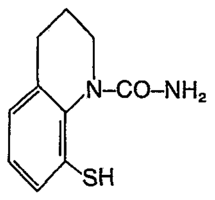

abgesaugt und mit 20 Teilen Äthylenglykol gewaschen. – Das glykolische Filtrat dient als Vorlage für den nächsten Ansatz einer hydrolytischen Ringöffnung von 3,4-Trimethylen-2-iminobenzthiazolin.

Der glykolfeuchte Filterrückstand wird in 300 Teile 10%ige Salzsäure eingetragen und die Mischung 60 Minuten unter Rühren bei 80 bis 85 °C erwärmt. Nach dem Abkühlen isoliert man das ausgefallene 3,4-Trimethylen-2(3H)-benzthiazolon der Formel

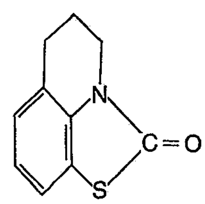

durch Filtration, wäscht den Rückstand mit Wasser neutral und trocknet ihn. Man erhält 62,8 Teile eines nahezu reinen Produktes mit einem Schmelzpunkt von 76 bis 77 °C entsprechend 65,8% d.Th. Es destilliert konstant bei 184 °C/4 mbar. Das Destillat schmilzt bei 77,5 bis 78 °C und ist analysenrein. – Wird die glykolische Reaktionsmischung entsprechend Beispiel 1 aufgearbeitet, so erhält man das 3,4-Trimethylen-2(3H)-benzthiazolon in entsprechender Reinheit mit einer Ausbeute von 89,8% d.Th.

Beispiel 7

Die Aus Beispiel 6 erhältliche glykolische Mutterlauge wird mit 95,0 Teilen 3,4-Trimethylen-2-iminobenzthiazolin und nur 20 Teilen Natriumhydroxid versetzt und 4 Stunden bei 160 bis 165 °C gerührt. Den Ansatz kühlt man sodann unter Rühren auf eine Temperatur von 20 bis 30 °C ab und verfährt anschliessend zur Cyclisierung und Aufarbeitung gemäss den Angaben des Beispieles 6.

Man erhält 86 Teile (90,1% d.Th.) 3,4-Trimethylen-2(3H)-benzthiazolon mit einem Schmelzpunkt von 77,5 bis 78 °C.

Beispiel 8

Man verfährt wie im Beispiel 6 und nachfolgend im Beispiel 7 angegeben, ersetzt jedoch die Ausgangsverbindung durch die äquivalente Menge an 3,4-Dimethyl-2-iminobenzthiazolin. Man erhält über die Zwischenstufe des Natriumsalzes des N-Methyl-N-(6-methyl-2-mercapto-phenyl)-harnstoffes als Endprodukt das 3,4-Dimethyl-2(3H)-benzthiazolon in einer Ausbeute von 69,3% gemäss der Arbeitsweise des Beispieles 6 und mit einer Ausbeute von 94,0% gemäss der Arbeitsweise des Beispieles 7, jeweils mit einem Schmelzpunkt von 123 bis 125 °C.

Beispiele 9 bis 42

Verfährt man in erfindungsgemässer Weise zur Herstellung von in 3-Stellung substituierten 2(3H)-Benzthiazolon-Verbindungen, beispielsweise in analoger Weise wie in den obigen Ausführungsbeispielen beschrieben und geht hierbei von Verbindungen entsprechend der allgemeinen Formel (1a) mit den in den nachfolgenden Tabellenbeispielen angegebenen Resten R, $R_1$ und $R_2$ aus, so erhält man die Verbindungen entsprechend der allgemeinen Formel (4a) mit den in diesen Tabellenbeispielen angegebenen Substituenten R, $R_1$ und $R_2$ mit den dort ebenfalls angegebenen Ausbeuten und Schmelzpunkten und/oder Siedepunkten:

| Bsp. | Verbindung (1a) bzw. (4a) R | $R_1$ | $R_2$ | Ausbeute (% d. Th.) | Smp. (°C) | Sdp. (°C/mbar) |
|---|---|---|---|---|---|---|
| 9 | Cyclohexyl | H | H | 85,3 | 72 | |
| 10 | Benzyl | H | H | 90,0 | 88 | 178/2,7 |
| 11 | $-(CH_2)_2-N(C_2H^5)_2$ | H | H | 81,9 | | 158/2,7 |
| 12 | β-(Piperidin-1-yl)-äthyl | H | H | 82,2 | | 171/1,3 |
| 13 | $-(CH_2)_3-N(C_2H_5)_2$ | H | H | 80,7 | | 152/4,0 |
| 14 | Phenyl | H | H | 92,2 | 82 | 227/21 |
| 15 | Methyl | 6-$CH_3$ | H | 91,5 | 77 | |
| 16 | Äthyl | 6-$CH_3$ | H | 84,0 | 58 | 132/1,3 |
| 17 | o-Methylphenyl | 4-$CH_3$ | H | 94,6 | 116 | 211/9,3 |
| 18 | p-Methylphenyl | 6-$CH_3$ | H | 93,0 | 106 | |
| 19 | Methyl | 5-$OCH_3$ | H | 84,2 | 107 | |
| 20 | Methyl | 6-$OC_2H_5$ | H | 90,8 | 85 | |
| 21 | Methyl | 4-F | H | 92,8 | 124 | |
| 22 | Methyl | 4-Cl | H | 96,1 | 132 | |
| 23 | Äthyl | 4-Cl | H | 88,7 | 99 | |
| 24 | n-Propyl | 4-Cl | H | 85,0 | 48 | 110/0,25 |
| 25 | n-Pentyl | 4-Cl | H | 83,0 | | 129/0,25 |
| 26 | Methyl | 5-Cl | H | 89,3 | 105 | |

| Bsp. | Verbindung (1a) bzw. (4a) R | $R_1$ | $R_2$ | Ausbeute (% d. Th.) | Smp. (°C) | Sdp. (°C/mbar) |
|---|---|---|---|---|---|---|
| 27 | Methyl | 6-Cl | H | 93,0 | 112 | |
| 28 | Benzyl | 6-Cl | H | 97,1 | 145 | |
| 29 | Methyl | 4-Br | H | 96,5 | 138 | |
| 30 | Methyl | 6-$NO_2$ | H | 89,0 | 162 | |
| 31 | Methyl | 4-$CH_3$ | 6-$CH_3$ | 91,4 | 151 | |
| 32 | Methyl | 4-$CH_3$ | 6-Cl | 92,7 | 131 | |
| 33 | Methyl | 4-$CH_3$ | 7-Cl | 87,0 | 129 | |
| 34 | Äthyl | H | H | 91,2 | | 152/5,7 |
| 35 | n-Propyl | H | H | 87,9 | | 130/1,3 |
| 36 | n-Butyl | H | H | 82,8 | | 150/4 |
| 37 | i-Amyl | H | H | 80,2 | | 144/4 |
| 38 | Äthyl | 6,7-Benzo | | 81,9 | 135 | 200/6,7 |
| 39 | o-Methylphenyl | 6,7-Benzo | | 96,0 | 118 | |
| 40 | p-Methylphenyl | 6,7-Benzo | | 94,8 | 171 | |
| 41 | p-Hydroxyphenyl | 6,7-Benzo | | 90,1 | 215 | |
| 42 | p-Methoxyphenyl | 6,7-Benzo | | 87,4 | 159 | |

**Patentanspruch**

1. Verfahren zur Herstellung von 2(3H)-Benzthiazolonen oder 2(3H)-Naphthothiazolonen, die in 3-Stellung jeweils mit einem gegebenenfalls substituierten Alkyl-, Cycloalkyl- oder Arylrest, die mit dem Benzol- oder Naphthalinkern verbunden sein können, substituiert sind, dadurch gekennzeichnet, dass man eine in 3-Stellung entsprechend substituierte 2(3H)-Iminobenzthiazolin- bzw. -naphthothiazolin-Verbindung mit einem Alkali- bzw. Erdalkalihydroxid in einem alkalibeständigen Lösungsmittel oder Verdünnungsmittel in Abwesenheit von Wasser oder lediglich in Anwesenheit von höchstens 2,5 Gew.-% Wasser behandelt und das sich so bildende Alkali- oder Erdalkalisalz der am Stickstoff substituierten ortho-Mercapto-N-phenyl- bzw. -naphthylharnstoff-Verbindung, gegebenenfalls ohne deren Zwischenisolierung, durch Behandlung mit einer Säure zur in 3-Stellung substituierten 2(3H)-Benzthiazolon- bzw. 2(3H)-Naphthothiazolon-Verbindung cyclisiert.

**Claim**

A process for the manufacture of 2(3H)-benzothiazolone compounds or of 2(3H)-naphthothiazolone compounds, which are each substituted in 3-position by an optionally substituted alkyl, cycloalkyl or aryl radical which radicals may be conjoint with the benzene or naphthalene nucleus, characterized by that a 2(3H)-iminobenzothiazoline compound substituted in 3-position correspondingly, or a 2(3H)-naphthothiazoline compound substituted in 3-position correspondingly, is treated with an alkali metal hydroxide or alkaline earth metal hydroxide in a solvent or diluent stable to alkalis, in the absence of water or merely in the presence of at most 2.5% by weight of water, and the alkali metal salt or alkaline earth metal salt of the ortho-mercapto-N-phenyl- or -naphthyl-urea compound substituted at the nitrogen atom, so formed, is cyclized, optionally without intermediate isolation, by treatment with an acid to give the 2(3H)-benzothiazolone compound substituted in 3-position, or the 2(3H)-naphthothiazolone compound substituted in 3-position.

**Revendication**

1. Procédé de préparation de 3H-benzothiazolones-2 ou de 3H-naphtothiazolones-2, substituées chacune en position 3 par un radical alkyle, cycloalkyle ou aryle, éventuellement substitué, qui peut être combiné au noyau benzène ou naphtalène, caractérisé en ce que l'on traite un composé 3H-iminobenzothiazoline-2 ou 3H-iminonaphtothiazoline-2 substitué d'une manière correspondante en position 3 avec un hydroxyde de métal alcalin ou alcalino-terreux dans un solvant ou un diluant résistant aux alcalis, en l'absence d'eau ou seulement en présence d'eau plus 2,5%-poids d'eau, et que l'on cyclise, par traitement avec un acide, pour obtenir le composé 3H-benzothiazolone-2 ou 3H-naphtathiazolone-2 substitué en position 3, le sel de métal alcalin ou alcalino-terreux, ainsi formé, du composé ortho-mercapto N-phényl urée ou ortho-mercapto N-naphtyl urée, éventuellement sans leur isolation intermédiaire.